## Europäisches Patentamt

## European Patent Office

## Office européen des brevets

(11) Veröffentlichungsnummer: **0 063 813**
B1

# EUROPÄISCHE PATENTSCHRIFT

(45) Veröffentlichungstag der Patentschrift:
02.01.85

(21) Anmeldenummer: 82103530.0

(22) Anmeldetag: 27.04.82

(51) Int. Cl.³: **C 07 C  43/04,** C 07 C  41/06,
C 07 C  41/42, C 07 C  31/12,
C 07 C  31/10, C 07 C  29/04,
C 07 C  29/86 // C07C11/06,
C07C11/09, C07C9/08,
C07C9/10, C07C9/12, C07C7/04,
C07C5/22, C07C5/333

(54) Verfahren zur Herstellung von Gemischen aus Isopropyl-tert.-butylether und tert.-Butylalkohol.

(30) Priorität: 28.04.81 DE 3116779

(43) Veröffentlichungstag der Anmeldung:
03.11.82 Patentblatt 82/44

(45) Bekanntmachung des Hinweises auf die Patenterteilung:
02.01.85 Patentblatt 85/1

(84) Benannte Vertragsstaaten:
AT BE CH DE FR GB IT LI LU NL SE

(56) Entgegenhaltungen:
CA - A - 958 213
US - A - 3 849 082
US - A - 4 046 520

(73) Patentinhaber: VEBA OEL AG,
Alexander-von-Humboldt-Strasse,
D-4650 Gelsenkirchen-Hassel (DE)

(72) Erfinder: Gottlieb, Klaus, Dr., Alte Strasse 59,
D-5804 Herdecke-Ende (DE)
Erfinder: Bruderreck, Hartmut, Dr.,
Bärenkampstrasse 55, D-4660 Gelsenkirchen-Buer (DE)
Erfinder: Wehmeier, Friedel-Heinrich, Dr.,
Klaus-Groth-Strasse 1, D-4250 Bottrop-Kirchhellen (DE)

(74) Vertreter: Krug, Joachim, Dr.,
Alexander-von-Humboldt-Strasse,
D-4650 Gelsenkirchen-Hassel (DE)

Die Akte enthält technische Angaben, die nach dem Eingang der Anmeldung eingereicht wurden und die nicht in dieser Patentschrift enthalten sind.

## Beschreibung

Die Erfindung betrifft ein Verfahren zur Herstellung von Isopropylalkohol, Isopropyl-tert.-butylether und tert.-Butylalkohol aus unter Normalbedingungen gasförmigen Kohlenwasserstoffen, die z. B. bei der Rohölförderung oder -verarbeitung anfallen. Das Sammeln, Reinigen, Transportieren und Verteilen dieser Paraffinkohlenwasserstoffe ist sehr aufwendig und mit hohen Kosten verbunden. Die Verwendung des $C_3$- und $C_4$-Anteiles dieser Paraffinkohlenwasserstoffe als Kraftstoffe in Verbrennungsmaschinen ist, obwohl technisch möglich, deswegen energetisch ungünstig. Die insgesamt für das Sammeln, Reinigen, Transportieren und Verteilen aufzuwendende Energie ist im Verhältnis zur in herkömmlichen Verbrennungsmotoren erzielten Energieausnutzung zu groß, so daß langfristig der Einsatz dieser Paraffinkohlenwasserstoffe als Autogas ohne steuerliche Begünstigung unwirtschaftlich ist.

Der vorliegenden Erfindung liegt die Aufgabe zugrunde, diese Paraffinkohlenwasserstoffe, die heute sogar noch in größeren Mengen abgefackelt werden, mit höherer Energierentabilität in wertvolle Kraftstoffkomponenten umzuwandeln, deren Erzeugung, Transport und Verteilung einen geringeren Energieeinsatz verlangt als Sammeln, Reinigen, Transport und Verteilung der gasförmigen Kohlenwasserstoffe und deren Einsatz in herkömmlichen Verbrennungsmotoren ohne Zusatzausrüstung möglich ist und dort zur Verringerung des spezifischen und absoluten Kraftstoffverbrauchs sowie zur Umweltentlastung beiträgt.

Aus den DE-OS 2 620 011 und 2 921 576 ist es bekannt, Butan zu Methyl-tert.-butylether zu verarbeiten. Hierbei wird n-Butan partiell oder vollständig zu Isobutan (2-Methyl-propan) isomerisiert und das n-Butan-Isobutan-Gemisch dehydriert, wobei neben Isobuten auch n-Butene, Butadien, Propen und Propan gebildet werden. Das Dehydrierungsreaktionsgemisch wird dann mit einem Überschuß an Methylalkohol veräthert, wobei sich das in der Dehydrierungsstufe gebildete Isobuten zu Methyl-tert.-butylether umsetzt. Der überschüssige Methylalkohol aus dem Verätherungsreaktionsgemisch wird entweder mit Wasser oder durch Azeotrop-Destillation entfernt. In der Isomerisierung und Dehydrierung werden von 25 bis zu 30 Gew.-% der eingesetzten Butanfraktion zu n-Buten, Butadien, Propan, Propen, Äthan, Äthen, Methan und Wasserstoff umgewandelt. Diese Isomerisier- und Dehydrierabgase können in den beschriebenen Verfahren nur als Brennstoff für Heizzwecke Verwendung finden, werden somit also erheblich abgewertet.

Hauptzweck des in DE-OS 2 921 576 beschriebenen Verfahrens ist die Erzeugung von reinem Methyl-tert.-butylether bzw. des in DE-OS 2 620 011 beschriebenen Verfahrens die Herstellung von reinem Methyl-tert.-butylether oder reinem Methyl-tert.-amylether, bzw. einem Gemisch dieser beiden reinen Äther aus Methanol und tert. Olefinen.

Aus den US-PS 3 904 384 und US-PS 4 046 520 ist es auch bekannt, Butane zu Isopropyl-tert.-butylether zu verarbeiten, wobei n-Butan isomerisiert und das erzeugte Isobutan nichtkatalytisch gecrackt wird, wobei neben Isobuten auch Propen gebildet wird. Das Propen wird zu Isopropanol und dieses zusammen mit dem gebildeten Isobuten zu Isopropyl-tert.-butylether umgewandelt. Auch diesem Verfahren haftet der große Nachteil an, daß ein großer Teil der eingesetzten Butanfraktion infolge des bei 640—670° C betriebenen Crackprozesses zu Produkten umgewandelt werden, die nur als Brennstoff für Heizzwecke eingesetzt werden können, wodurch die Gesamtenergierentabilität wiederum erheblich beeinträchtigt wird. Auch soll nach diesem Verfahren wiederum nur reiner Isopropyl-tert.-butylether hergestellt werden.

Im Gegensatz hierzu ist das Ziel vorliegender Erfindung, nicht Reinkomponenten, sondern Gemische aus gegebenenfalls Alkohol enthaltendem Äther zu erzeugen, die in bestimmten Zusammensetzungen synergistische Effekte bei der Verbrennung im Motor zeigen und die, wie Experimente und Berechnungen zur Energienutzung aller eingesetzten und erzeugten Stoffe gezeigt haben, hinsichtlich des Gesamtenergieverbrauchs optimiert sind.

Hierzu soll nach der vorliegenden Erfindung die Umwandlung auch des bei der Isomerisierung und Dehydrierung anfallenden Propens und Propans sowie auch des in den Einsatzkohlenwasserstoffen enthaltenen Propans zu $C_3$-Alkohol und mit Isobuten zu dem auf $C_3$-Alkohol aufbauenden Äther erfolgen, wobei auch ein Teil des Isobutens mit Wasser zu tert.-Butylalkohol verarbeitet wird.

Hierfür werden gemäß vorliegender Erfindung aus dem Propan und Butan enthaltenden Gemisch leichter Kohlenwasserstoffe eine Propan- und eine Butanfraktion abgetrennt. Propan wird katalytisch dehydriert und das entstandene Propen mit Wasser zu Isopropylalkohol (Propan-2-ol) umgesetzt. In der Butanfraktion wird das n-Butan isomerisiert, katalytisch dehydriert und das im Dehydrierungsreaktionsgemisch enthaltene Isobuten zumindest teilweise mit einem Gemisch des aus der Propanfraktion gewonnenen Isopropylalkohols und Wasser zu einem Gemisch aus tert.-Butylalkohol und Isopropyl-tert.-butylether umgesetzt. Die nicht umgesetzten Kohlenwasserstoffe aus der Propen-Hydratisierung sowie nicht umgesetztes Isobutan aus der Verätherung werden in die Dehydrierstufe zurückgeführt.

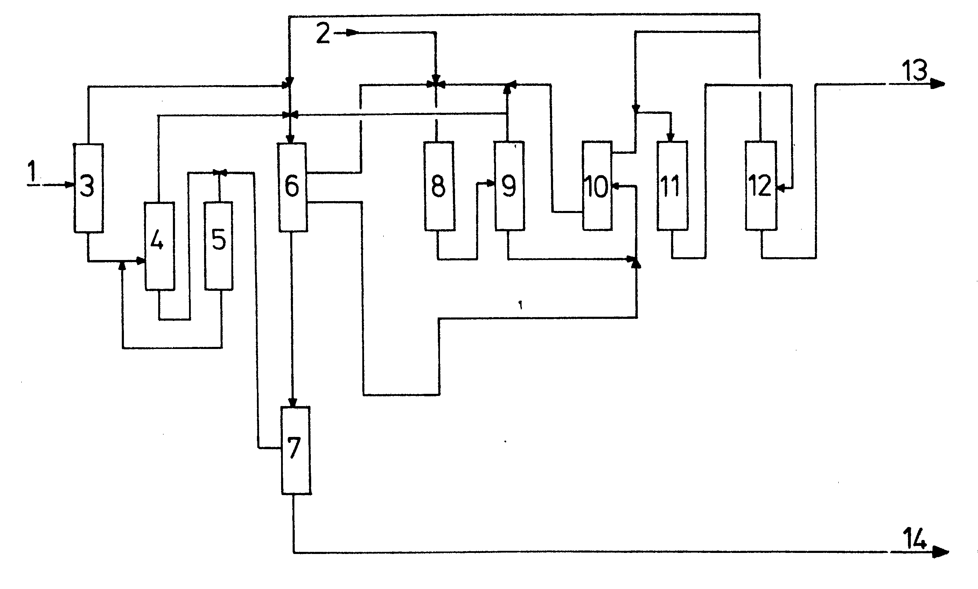

Eine Ausbildung der Erfindung ergibt sich aus der nachfolgenden Verfahrensbeschreibung, in der das Verfahren unter Bezugnahme auf die in der zugehörigen Zeichnung enthaltene Prinzipskizze näher erläutert wird. Die Zeichnung stellt eine bevorzugte Ausführungsform des erfindungsgemäßen Verfahrens unter Weglassung der für das Verständnis nicht erforderlichen Teile wie Pumpen, Wärmetauscher und einiger Destillationskolonnen dar.

Das Kohlenwasserstoffgemisch 1 wird durch Destillation 3 in eine Butan und eine Propan enthaltende Fraktion aufgetrennt. Die Isomerisierung 5 — n-Butan zu Isobutan — erfolgt in an sich bekannter Weise an einem Platin-haltigen Festbettkatalysator in Gegenwart von Wasserstoff bei Temperaturen von 150—210°C und Drücken von 15—30 bar. Die Reaktionsbedingungen hinsichtlich Druck und Temperatur werden insgesamt so eingestellt, daß das Isomerisierungsgleichgewicht möglichst erreicht wird.

Von dem den Isomerisierungsreaktor 5 verlassenden, zu mehr als 50% aus Isobutan bestehenden Reaktionsgemisch werden Wasserstoff und bei der Isomerisierung entstandenes Methan, Äthan und Propan abgetrennt, das $C_4$-Isomerisat wird mit der eingesetzten Butanfraktion vereinigt. Ein aliquoter Teil des Isobutan-n-Butan-Gemisches wird in einer Destillationskolonne 4 mit 30—100 Böden unter einem Druck von 7—14 bar und bei Temperaturen von 40—80°C rektifiziert, so daß das über Kopf entnommene Isobutan-n-Butan-Gemisch einen Gehalt von 95—99% Isobutan aufweist. Das dem Sumpf der Destillationskolonne 4 entnommene n-Butan wird in die Isomerisierung 5 zurückgeführt. Das Kopfprodukt der Destillationskolonne 4 wird zusammen mit dem rückgeführten Isobutan aus der Verätherung 11 der Dehydrierung 6 zugeführt.

Der Propanstrom kann mit dem aus der Hydratisierung 8 rückgeführten Propan vereinigt und einer getrennten Dehydrierstufe zugeführt werden. In der bevorzugten Ausführungsform des erfindungsgemäßen Verfahrens wird die Propanfraktion mit den aus der Hydratisierung 8 rückgeführten Kohlenwasserstoffen vereinigt und zusammen mit der Isobutan-Beschickung der gemeinsamen Dehydrierung 6 zugeführt.

Die Dehydrierung der $C_4$- und $C_3$-Kohlenwasserstoffe erfolgt in an sich bekannter Weise katalytisch, wahlweise in einem Festbett- oder einem Fließbettreaktor. Die Dehydriertemperatur liegt zwischen 530 und 700°C, der Druck zwischen 0,2 und 5 bar, vorzugsweise zwischen 0,3 und 1,5 bar. Der Dehydrierungskatalysator besteht im allgemeinen aus aktivem Aluminiumoxid und Zusätzen aus Chromoxid oder Platin, die durch Tränken auf $Al_2O_3$ aufgebracht werden.

Der während der Reaktionsphase entstehende Koks wird in einer Regenerierphase mit Luft abgebrannt, die dabei freiwerdende Wärme wird als Prozeßwärme zurückgewonnen. Die Dehydrierungsreaktionsgemische werden durch Abkühlen und Komprimieren in einen gasförmigen, vorwiegend Methan, Äthan, Äthylen und den Wasserstoff enthaltenden Strom und flüssige, vorwiegend Propan, und Propen bzw. Isobutan und Isobuten enthaltende Ströme aufgetrennt.

Aus dem gasförmigen Strom wird der Wasserstoff in der Reinigungsanlage 7 in an sich bekannter Weise weitgehend abgetrennt. Wenn keine adäquate Verwendungsmöglichkeit für den Gesamtwasserstoff besteht, wird der Methan- und Äthan-haltigen Fraktion des Reaktionsgemisches nach Dehydrierung nur so viel Wasserstoff entnommen, wie für die Isomerisierungs- und Hydrierungsreaktionen notwendig ist. Der Restwasserstoff kann mit dem Dehydrierabgas bei 14 zur Prozeßenergieerzeugung entnommen werden.

Wird nach der bevorzugten Ausführungsform des erfindungsgemäßen Verfahrens mit gemeinsamer Dehydrierung 6 von Propan und Butan gearbeitet, so wird das die $C_3$- und $C_4$-Kohlenwasserstoffe enthaltende Dehydrierungsreaktionsgemisch in einer Rektifizierungskolonne in einen Propan- und Propen-haltigen Strom sowie einen vorwiegend Isobutan und Isobuten enthaltenden Strom aufgetrennt.

Die aus dem Dehydrierungsgemisch abgetrennte Propen-haltige $C_3$-Fraktion wird dem Hydratisierungsreaktor 8 zugeführt, in dem durch katalytische Synthese von Propen und Wasser 2 bei einem Druck von 30—100 bar, vorzugsweise bei 40—80 bar, und Temperaturen von 100—170°C, vorzugsweise 130—160°C, Isopropylalkohol hergestellt wird. Als Katalysatoren dienen saure Katalysatoren, bevorzugt stark saure Kationenaustauscher, die aus sulfonierten, mit Divinylbenzol vernetzten Polystyrolharzen bestehen.

Im Beschickungsstrom werden auf 1 Mol Propen 1—20 Mole Wasser, vorzugsweise 3—8 Mole, eingesetzt. Die Raumgeschwindigkeit in Liter Beschickung pro Liter Katalysator und Stunde beträgt 0,3—25, vorzugsweise 0,5—10. Unter diesen Reaktionsbedingungen werden 10—70% des eingesetzten Propens unter Bildung von Isopropylalkohol und Diisopropylether umgesetzt.

Von dem Reaktionsendgemisch werden durch Destillieren die $C_3$-Kohlenwasserstoffe über Kopf der Destillierkolonne 9 abgetrennt; ein Teilstrom wird in den Hydratisierungsreaktor 8 und ein mengenmäßig kleinerer Teil in die Dehydrierung zurückgeführt. Das Isopropylalkohol-Wassergemisch wird, gegebenenfalls nach destillativer Anreicherung von Isopropylalkohol, mit einem als Extraktionsmittel für Isopropylalkohol geeigneten, mit Wasser nicht mischbaren und von Isopropylalkohol leicht abtrennbaren organischen Lösungsmittel vermischt. Es ist ein besonderes Merkmal dieser Erfindung, daß hierfür der im Verfahren erzeugte olefinhaltige $C_3$- oder insbesondere $C_4$-Strom verwendet wird.

Nach Trennung des Extraktionsgemisches in eine organische Phase und eine wäßrige Phase enthält die organische Phase 40—95% der entstandenen Isopropylalkoholmenge 80—98% der Di-isopropylethermenge und Wasser. Von der organischen Phase werden durch Destillation ein Teil der Kohlenwasserstoffe abgetrennt und in die Extraktion 10 zurückgeführt. Das dem Sumpf entnommene Isopropylalkohol-Wassergemisch wird einschließlich des gebildeten Di-isopropylethers der Verätherung 11 zugeführt.

In der bevorzugten Ausführungsform wird zu Extraktion 10 die Isobuten enthaltende $C_4$-Fraktion

eingesetzt. Dazu wird ein Gewichtsteil des aus dem Sumpf der Kolonne 12 abgezogenen Wasser-Isopropylalkohol-Gemisches mit 1—20 Gewichtsteilen der $C_4$-Fraktion vermischt und der Extraktionsstufe 10 zugeführt, wo das Gesamtgemisch in eine wäßrige und eine organische Phase aufgetrennt wird. Die organische Phase enthält 20—60 Gew.-% des der Extraktion zugeführten Isopropylalkohols und Wasser; hieraus wird durch Destillieren ein Teil der Isobuten enthaltenden $C_4$-Fraktion abgetrennt, so daß der Rückstand neben Wasser Isobuten und Isopropylalkohol in dem für die Verätherung 11 notwendigen stöchiometrischen Verhältnis enthält. Die wäßrige Phase wird in die Hydratisierung 8 rückgeführt.

Um die Trennleistung der Extraktionsstufe zu erhöhen, kann aus dem am Sumpf der Kolonne 9 abgezogenen Isopropylalkohol-Wasser-Gemisch durch Destillieren zunächst ein an Isopropylalkohol angereichertes Isopropylalkohol-Wasser-Gemisch abgetrennt und wie vorgehend beschrieben mit der Isobuten-enthaltenden $C_4$-Fraktion behandelt werden. Der Anreicherungsgrad kann bis zu 88 Gew.-% betragen. Zur extraktiven Abtrennung eines Isopropylalkohol-Wassergemischs werden dann ein Gewichtsanteil des an Isopropylalkohol angereicherten wäßrigen Gemisches mit 1—5Gewichtsteilen Isobuten enthaltender $C_4$-Fraktion gemischt und der Extraktionsstufe 10 zugeführt, wo eine 70—95 Gew.-% des in der Hydratisierung gebildeten Isopropylalkohols und Wasser enthaltende organische Phase abgetrennt wird. Restwasser wird wieder in die Hydratisierung zurückgeführt.

In einer besonderen Ausführungsform des erfindungsgemäßen Verfahrens kann ein nach der Hydratisierung 8 über Kopf der Anreicherungskolonne abgenommenes Isopropylalkohol-Wassergemisch direkt der Verätherung 11 zugeführt und das tert.-Butylalkoholhaltige Äther-Alkoholgemisch hergestellt und bei 13 abgezogen werden.

Aus der DE-OS 2 535 471 sowie US-PS 4 046 520 und CA-PS 958 213 ist die Herstellung von Isopropyl-tert.-butylether an sich bekannt. Im Unterschied zu den dort beschriebenen Ausführungsformen, die alle auf einen während der Reaktion vorhandenen Überschuß an Isopropylalkohol und damit notwendigerweise auf höhere Temperaturen abheben, wird in der vorliegend beschriebenen bevorzugten Ausführungsform mit einem Überschuß an Isobuten und niedrigeren Temperaturen gearbeitet, wodurch ein höherer Alkohol-Umsatz erreicht wird und somit auf eine Abtrennung und Rückführung des nicht umgesetzten Alkohols verzichtet werden kann.

Ein weiterer Vorteil der Arbeitsweise mit niedrigen Reaktionstemperaturen ist, daß eine Dehydratisierung des Isopropylalkohols zu Wasser und Propen vermieden wird, was die Wirtschaftlichkeit der Verfahren bei höheren Reaktionstemperaturen nachteilig beeinflußt.

Die Isobuten-haltige $C_4$-Fraktion wird mit dem erfindungsgemäß hergestellten Isopropylalkohol-Wassergemisch in Gegenwart saurer Katalysatoren umgesetzt, wobei Alkohol zu 10—95%, vorzugsweise zu 50—90%, unter Bildung von Isopropyl-tert.-butylether und Wasser zu 80—100%, unter Bildung von tert.-Butylalkohol mit Isobuten umgesetzt werden. Das als Beschickung verwendete Alkohol-Wassergemisch kann 1—50 Gew.-% Wasser enthalten, insbesondere kann man ein bei einer Azeotrop-Destillation, z. B. das bei der beschriebenen Isopropylalkohol-Anreicherung anfallende Isopropylalkohol-Wasser-Gemisch einsetzen. Als saure Katalysatoren dienen sulfonierte Kationenaustauscherharze, bevorzugt wiederum stark saure Ionenaustauscher auf der Basis von sulfonierten, mit Divinylbenzol vernetzten Polystyrol.

Es wurde überraschenderweise gefunden, daß auch in Gegenwart von Wasser die n-Butene keine Umsetzung eingehen. Überraschenderweise wurde weiterhin festgestellt, daß tert.-Butylalkohol kein Reaktionsprodukt durch Parallelreaktion mit Isobuten bildet. Die Verätherung erfolgt in einem mehrstufigen Festbettreaktor bei Temperaturen zwischen 20 und 150°C, vorzugsweise bei 30—80°C und Drücken, die zur Verflüssigung von Isobuten ausreichen, also von 4—40 bar, vorzugsweise 8—16 bar. Das Molverhältnis von Isopropylalkohol zu Isobuten liegt im Bereich von 0,1 : 1 bis 1 : 10, vorzugsweise von 1 : 0,7 bis 1 : 5, das Molverhältnis von Wasser zu Isobuten im Bereich von 1 : 1 bis 1 : 20, vorzugsweise von 1 : 1,5 bis 1 : 10, die Raumgeschwindigkeit in Liter Beschickungsprodukt pro Liter Katalysator und Stunde im Bereich von 0,3—50, vorzugsweise 1—20. Das Äther-Alkohol-Gemisch wird durch Destillation unter Druck von den nicht umgesetzten Kohlenwasserstoffen abgetrennt, welche ihrerseits in die Verätherung rückgeführt werden.

Die an sich bekannte Herstellung von tert.-Butylalkohol aus Isobuten und Wasser an sauren Ionenaustauschern zusammen mit der Herstellung von Äther aus Isobuten und Isopropylalkohol im gleichen Reaktionsschritt ist bisher noch nicht beschrieben. Der besondere Wert dieses Verfahrens beruht darauf, daß bei sehr niedrigen Temperaturen und einem auf Wasser bezogenen Isobutenüberschuß ein nahezu 100prozentiger Umsatz von Wasser zu tert.-Butalalkohol, ohne Bildung nennenswerter Mengen an Isobutanoligomeren erzielt wird. In den beschriebenen Verfahren von tert.-Butylalkohol muß mit einem auf Isobuten bezogenen Überschuß an Wasser gearbeitet werden, um den Zwangsfall an Isobutenoligomeren niedrig zu halten. Um eine ausreichende Ausbeute an tert.-Butylalkohol zu erreichen, muß auch bei hohen Temperaturen gearbeitet werden. Die Herstellung von wasserfreiem tert.-Butylalkohol nach den bereits bekannten Verfahren ist zudem mit hohen Trennkosten verbunden.

Das erfindungsgemäße Äther-Alkohol-Gemisch wird aus dem Sumpf der Destillationskolonne 12 bei 13 entnommen. Soll ein Isopropanol-freies Isopropyl-tert.-butylether-tert.-Butylalkohol-Gemisch erfindungsgemäß hergestellt werden, so kann ein an Isopropylalkohol angereicherter Teil des Äther-Alkohol-Gemischs aus dem Sumpf der Kolonne 12 abgetrennt und in die Verätherung rückgeführt werden.

## Beispiel 1

Ein 0,98 Mol Wasser und 1,75 Mol Isopropylalkohol enthaltendes Gemisch wurde mit Isobuten im molaren Mengenverhältnis Wasser zu Isopropylalkohol zu Isobuten = 0,98 : 1,75 : 2,60 bei einer Temperatur von 50° C und einem Druck, über dem Dampfdruck von Isobuten, so daß dieses als Flüssigkeit vorliegt, nämlich 16 bar, umgesetzt. Der mit Katalysator gefüllte Reaktor wurde mit 3,8 Gewichtsteilen genannten Gemischs aus Wasser, Isopropylalkohol und Isobuten pro Stunde und pro Gewichtsteil getrocknetem Katalysator beschickt. Als Reaktor wurde ein schlanker Rohrreaktor mit einem Verhältnis von Innendurchmesser zu Länge von 1 : 30 und als Katalysator ein stark saures Ionenaustauscherharz (Handelsprodukt Lewatit SPC 118) verwendet. Zur Einstellung der genannten Temperatur wurde ein geeigneter Vorheizer verwendet; die bei der Reaktion freiwerdende Wärme wurde über einen Kühler abgeführt. Das Reaktionsgemisch wurde durch Destillieren von nicht umgesetztem Isobuten weitgehend befreit und hatte die in Tabelle 1 angegebene Zusammensetzung.

Tabelle 1

| Komponente | Gew.-% |
|---|---|
| Isobuten | 0,9 |
| Isopropylalkohol | 24,4 |
| tert.-Butylalkohol | 30,6 |
| Isopropyl-tert.-butylether | 43,1 |
| Isobutenoligomere | 0,5 |
| Wasser | 0,4 |

## Beispiel 2

Ein 0,83 Mol Wasser und 1,42 Mol Isopropylalkohol enthaltendes Gemisch wurde mit einem Isobuten enthaltendem $C_4$-Kohlenwasserstoffgemisch der in Tabelle 2, Spalte 1 angegebenen Zusammensetzung im molaren Mengenverhältnis Wasser zu Isopropylalkohol zu Isobuten = 0,83 : 1,42 : 3,58 in einem aus zwei in Serie geschalteten Reaktoren bestehendem Reaktorsystem umgesetzt. Der erste Reaktor war ein schlanker Rohrreaktor mit einem Verhältnis von Innendurchmesser zu Länge von 1 : 30, der zweite Reaktor hatte ein Verhältnis von Innendurchmesser zu Länge von 1 : 10. Als Katalysator wurde ein stark saures Ionenaustauscherharz (Handelsprodukt Lewatit SPC 118) verwendet. Die Temperatur am Eintritt des ersten Reaktors betrug 70° C, am Eintritt des zweiten Reaktors 40° C. Der Druck war über dem Dampfdruck von Isobuten, so daß das $C_4$-Kohlenwasserstoffgemisch als Flüssigkeit vorlag, nämlich 16 bar, gewählt.

Das mit Katalysator befüllte Reaktorsystem wurde pro Stunde und pro Gewichtsteil getrockneten Katalysators mit 10,1 Gewichtsteilen genannten Gemisches aus Wasser, Isopropanol und Isobuten enthaltenden $C_4$-Kohlenwasserstoffen beschickt. Das Verhältnis des Katalysatorvolumens in Reaktor 1 zum entsprechenden Volumen in Reaktor 2 betrug 1 : 3. Zur Einstellung der genannten Temperaturen wurde vor dem ersten Reaktor ein geeigneter Vorheizer, nach dem ersten bzw. vor dem zweiten Reaktor ein geeigneter Kühler verwendet. Die Reaktionswärme wurde ebenfalls durch einen geeigneten Kühler abgeführt.

Das Reaktionsendgemisch wurde durch Destillation von den nicht umgesetzten Kohlenwasserstoffen weitgehend befreit und hatte die in Tabelle 2, Spalte 2 angegebene Zusammensetzung. Die Zusammensetzung der nicht umgesetzten Kohlenwasserstoffe hatte die in Spalte 3 der Tabelle angegebene Zusammensetzung.

Tabelle 2

| Komponente | Gew.-% 1 | Gew.-% 2 | Gew.-% 3 |
|---|---|---|---|
| Isobutan | 40,5 | 0,2 | 55,4 |
| n-Butan | 2,1 | — | 3,0 |
| n-Buten-1 | 2,5 | — | 3,4 |
| n-Buten-2 | 4,8 | — | 6,8 |
| Isobuten | 50,1 | 0,3 | 31,4 |
| Isopropylalkohol | — | 8,8 | — |
| tert.-Butylalkohol | — | 29,0 | — |
| Isobutenoligomere | — | 0,1 | — |
| Isopropyl-tert.-butylether | — | 61,5 | — |
| Wasser | — | 0,1 | — |

**Patentansprüche**

1. Verfahren zur Herstellung eines gegebenenfalls Isopropylalkohol enthaltenden Gemisches von Isopropyl-tert.-butylether und tert.-Butylalkohol aus einem Gemisch leichter Kohlenwasserstoffe, die Propan und Butan enthalten, dadurch gekennzeichnet, daß eine Propan- und eine Butanfraktion abgetrennt werden, n-Butan isomerisiert wird, Butan und Propan katalytisch dehydriert werden, Propen mit Wasser zu Isopropylalkohol umgesetzt und ein Isopropylalkohol-Wassergemisch mit dem im Dehydrierungsreaktionsgemisch enthaltenen Isobuten unter Bildung von Isopropyl-tert.-butylether und tert.-Butylalkohol umgesetzt wird, während nicht umgesetzte Kohlenwasserstoffe aus der Propen-Hydratisierung sowie nicht umgesetztes Isobutan aus der Verätherung in die Dehydrierstufe zurückgeführt werden.

2. Verfahren nach Anspruch 1, dadurch gekennzeichnet, daß die Propanfraktion gemeinsam mit der Butanfraktion dehydriert und das erhaltene Dehydrierungsreaktionsgemisch wieder in eine C$_3$-Fraktion und eine C$_4$-Fraktion getrennt wird.

3. Verfahren nach Anspruch 1 oder 2, dadurch gekennzeichnet, daß ein Isopropylalkohol-Wasser-Gemisch in Gegenwart saurer Katalysatoren mit flüssigem Isobuten bei Temperaturen von 20—150, vorzugsweise 30—80°C zu tert.-Butylalkohol und Isopropyl-tert.-butylether umgesetzt wird, wobei eine solche Isobutenmenge eingesetzt wird, daß auf 1 Mol Wasser 1—20 Mole Isobuten und auf 1 Mol Isopropylalkohol 0,1—10 Mole Isobuten entfallen.

4. Verfahren nach einem der Ansprüche 1 bis 3, dadurch gekennzeichnet, daß durch Rektifizieren aus dem Reaktionsgemisch der Hydratisierung ein Isopropylalkohol-Wasser-Gemisch abgetrennt, aus diesem durch Behandeln mit einer Isobuten enthaltenden C$_4$-Fraktion Wasser enthaltender Isopropylalkohol extrahiert, daraus ein Isopropylalkohol und Isobuten sowie Wasser enthaltendes Gemisch abdestilliert und der Verätherung zugeführt wird, während die von Isopropylalkohol befreite C$_4$-Fraktion in die Extraktion und die wäßrige Phase aus der Extraktion in die Hydratisierung zurückgeführt werden.

5. Verfahren nach einem der Ansprüche 1 bis 4, dadurch gekennzeichnet, daß das Isomerisat der Butanfraktion in eine n-Butan- und eine Isobutanfraktion getrennt, die n-Butanfraktion in die Isomerisierung zurückgeführt wird, die Isobutanfraktion einschließlich des nach der Verätherung rückgeführten Isobutans und einschließlich der aus der Hydratisierungsstufe zurückgeführten Kohlenwasserstoffe dehydriert wird.

**Claims**

1. Process for producing a mixture of isopropyl tert-butyl ether and tert-butyl alcohol, which mixture may contain isopropyl alcohol, from a mixture of light hydrocarbons containing propane and butane, characterized in that a propane and a butane fraction are separated, n-butane is isomerized, butane and propane are catalytically dehydrogenated, propene is converted with water into isopropyl alcohol and a mixture of isopropyl alcohol and water is reacted with the isobutene contained in the dehydrogenation reaction mixture thereby forming isopropyl tert-butyl ether and tert-butyl alcohol, while unconverted hydrocarbons from the propene hydration and unconverted isobutane from the etherification are recycled to the dehydrogenation stage.

2. Process according to claim 1, characterized in that the propane fraction together with the butane fraction is dehydrogenated and the resulting dehydrogenation reaction mixture is again separated into a $C_3$-fraction and a $C_4$-fraction.

3. Process according to claim 1 or 2, characterized in that a mixture of isopropyl alcohol and water is reacted with liquid isobutene in the presence of acid catalysts at temperatures of 20 to 150, preferably 30 to 80° C to form tert-butyl alcohol and isopropyl tert-butyl ether, wherein as much isobutene is used as is needed to provide 1 to 20 moles of isobutene per 1 mole of water and 0.1 to 10 moles of isobutene per 1 mole of isopropyl alcohol.

4. Process according to one of the claims 1 to 3, characterized in that a mixture of isopropyl alcohol and water is separated from the hydration reaction mixture by rectification, isopropyl alcohol containing water is extracted from this mixture by the treatment with a $C_4$-fraction containing isbutene, a mixture containing isopropyl alcohol and isobutene and possibly water is distilled off from this extract and fed to the etherification, while the $C_4$-fraction freed of isopropyl alcohol is recycled to the extraction and the aqueous phase from the extraction is recycled to the hydration.

5. Process according to one of the claims 1 to 4, characterized in that the isomerizate of the butane fraction is divided into a n-butane und an isobutane fraction, the n-butane fraction is recycled to the isomerization, the isobutane fraction, including the isobutane recycled after the etherification and including the hydrocarbons which are recycled from the hydration stages, is dehydrogenated.


## Revendications

1. Procédé pour la fabrication d'un mélange contenant éventuellement de l'alcool isopropylique, de tert.-butyléther d'isopropyle et d'alcool butylique tert.-, à partir d'un mélange d'hydrocarbures légers qui contiennent du propane et du butane, procédé caractérisé en ce que l'on sépare une fraction propane et une fraction butane, isomérise le n-butane, déshydrogènise catalytiquement le butane et le propane, fait réagir le propène avec de l'eau en alcool isopropylique, et fait réagir un mélange d'alcool isopropylique et d'eau avec l'isobutène contenu dans le mélange réactionnel de la déshydrogénation, avec formation de tert.-butyléther d'isopropyle et d'alcool butylique tert., pendant que les hydrocarbures qui n'ont pas réagi, provenant de l'hydratation du propène, ainsi que l'isobutane qui n'a pas réagi, provenant de l'éthérification sont retournés dans l'étage de déshydrogénation.

2. Procédé suivant la revendication 1, caractérisé en ce que la fraction propane est déshydrogénisée en commun avec la fraction butane et que le mélange réactionnel de déshydrogénation est à nouveau séparé en une fraction en $C_3$ et une fraction en $C_4$.

3. Procédé suivant l'une des revendications 1 ou 2, caractérisé en ce que l'on fait réagir un mélange d'alcool isopropylique et d'eau, en présence de catalyseurs acides, avec de l'isobutène liquide, à des températures de 20 à 150, de préférence 30 à 80°C en alcool butylique tert. et en tert.-butyléther d'isopropyle, la quantité d'isobutène mise en œuvre étant telle que l'on trouve, pour 1 mol d'eau, 1 à 20 mol d'isobutène, et pour 1 mole d'alcool isopropylique, 0,1 à 10 mol d'isobutène.

4. Procédé suivant l'une des revendications 1 à 3, caractérisé en ce que l'on sépare, par rectification du mélange réactionnel de l'hydratation, un mélange alcool isopropylique-eau, extrait de ce dernier, par traitement avec une fraction en $C_4$ contenant de l'isobutène, de l'alcool isopropylique contenant de l'eau, en retire par distillation un mélange contenant de l'alcool isopropylique et de l'isobutène ainsi que de l'eau et l'envoie à l'éthérification, pendant que la fraction en $C_4$, d'où on a retiré l'alcool isopropylique, est retournée à l'extraction, et la phase aqueuse provenant de l'extraction, est retournée à l'hydratation.

5. Procédé suivant l'une des revendications 1 à 4, caractérisé en ce que l'isomérisat de la fraction butane est séparé en une fraction n-butane et une fraction isobutane, que la fraction n-butane est retournée à l'isomérisation, et la fraction isobutane, y compris l'isobutane retourneé après l'éthérification et y compris les hydrocarbures retournés de l'étape d'hydratation est déshydrogéné.